# EUROPÄISCHE PATENTANMELDUNG

(11) **EP 3 524 678 A1**
(43) Veröffentlichungstag der Anmeldung: **14.08.2019**
(21) Anmeldenummer: 19163943.4
(22) Anmeldetag: 08.12.2006
(51) Int. Cl.: C12N 15/10, A61K 31/7088

(54) **VERFAHREN ZUR ANREICHERUNG VON KURZKETTIGEN NUKLEINSÄUREN**

(30) Priorität: 09.12.2005 DE 102005059315
(62) Teilanmeldung aus: 16157978.4
(71) Anmelder: QIAGEN GmbH, 40724 Hilden (DE)
(72) Erfinder: RITT, Christoph, 40724 Hilden (DE); HIMMELREICH, Ralf, 55131 Mainz (DE); WEBER, Martin, 1170 Wien (AT)
(74) Vertreter: Roth, Carla

(57) **Zusammenfassung**

Die vorliegende Erfindung betrifft ein Verfahren zur Anreicherung von Nukleinsäuren mit einer Länge von weniger als 300 Nukleotiden, umfassend die Verfahrensschritte
i) Bereitstellen einer fluiden, vorzugsweise wässrigen, Phase P₁ beinhaltend
(α1) mindestens eine Nukleinsäure mit einer Länge von weniger als 300 Nukleotiden, sowie
(α2) mindestens eine von dieser Nukleinsäure (α1) verschiedene Komponente,

ii) in Kontakt bringen der Phase P₁ mit einer Anionenaustausch-Matrix zum Anbinden der Nukleinsäure (α1) an die Anionenaustausch-Matrix,
iii) gegebenenfalls Waschen der Anionenaustausch-Matrix mit einem Waschpuffer, wobei die Nukleinsäure (α1) an der Anionenaustausch-Matrix gebunden bleibt, sowie
iv) Abtrennen, vorzugsweise Eluieren, der an der Anionenaustausch-Matrix gebundenen Nukleinsäure (α1) von der Anionenaustausch-Matrix unter Erhalt einer fluiden, vorzugsweise wässrigen, Phase P₂ beinhaltend die Nukleinsäure (α1).

Die Erfindung betrifft auch einen Kit zur Anreicherung von Nukleinsäuren mit einer Länge von weniger als 300 Nukleotiden, die Verwendung dieses Kits, die Verwendung einer Anionenaustausch-Matrix sowie ein Verfahren zur Behandlung einer Krankheit.

## Beschreibung

Die vorliegende Erfindung betrifft ein Verfahren zur Anreicherung von Nukleinsäuren mit einer Länge von weniger als 300 Nukleotiden, einen Kit zur Anreicherung von Nukleinsäuren mit einer Länge von weniger als 300 Nukleotiden, die Verwendung dieses Kits, die Verwendung einer Anionenaustausch-Matrix sowie ein Verfahren zur Behandlung einer Krankheit.

Seit vor wenigen Jahren entdeckt wurde, dass kleine Ribonukleinsäuren (RNAs) eine wesentliche regulatorische Funktion bei der Genexpression übernehmen, treten kleine RNAs, die kürzer als 300 Nukleotide, insbesondere kürzer als 100 Nukleotide sind, immer mehr in den Focus wissenschaftlicher Untersuchungen. Insbesondere die Micro-RNAs (miRNAs) haben die Aufmerksamkeit vieler Forscher geweckt. miRNAs sind eine evolutionär konservierte Klasse kleiner nicht kodierender RNAs von etwa 22 Nukleotiden Länge, deren komplexe Rolle bei der Regulation der Genexpression mehr und mehr offenbar wird. miRNAs wurden in jedem untersuchten eukaryotischen Organismus und in beinahe jedem Gewebe gefunden, so unter anderem in Pilzen, Pflanzen, Insekten und Säugern. Neben den miRNAs wurden noch weitere kleine RNAs entdeckt, die ebenfalls eine fundamentale Rolle bei den zellulären Funktionen spielen. Zu diesen gehören z.B. small interfering RNAs (siRNAs), small nuclear RNAs (snRNAs) und small nucleolar RNAs (snoRNAs).

Um die zelluläre Rolle dieser kurzen RNAs, die kürzer als 300 Nukleotide sind, untersuchen zu können, müssen diese möglichst rein und mit hoher Ausbeute aus den zu untersuchenden biologischen Systemen aufgereinigt werden. Es besteht daher ein großes Bedürfnis, ein Verfahren anzugeben, mit denen solche kurzen RNAs aus komplexen biologischen Systemen, insbesondere aus Zelllysaten, aufgereinigt bzw. isoliert werden können.

Um allgemein Nukleinsäuren aus biologischen Proben zu isolieren, müssen diese von den übrigen zellulären Bestandteilen, wie Proteinen, Zuckern, Lipiden und weiteren Komponenten abgetrennt werden. Aus dem Stand der Technik sind viele Methoden zur Abtrennung von Nukleinsäuren aus den unterschiedlichsten Ausgangsmaterialien, wie etwa aus Zellkulturen, aus Geweben pflanzlichen und tierischen Ursprungs sowie aus Körperflüssigkeiten, bekannt. Eine Methode beinhaltet beispielsweise die Extraktion der in der Regel wässrigen Ausgangslösungen mit Hilfe von organischen Lösungsmitteln, wie Phenol und Chloroform (Chomczynski und Sacchi, 1987), gefolgt von einer Präzipitation der Nukleinsäuren mit Hilfe von Alkoholen, wie Ethanol oder Isopropanol, aus der wässrigen Phase (Sambrook, J., Fritsch, E. F. in T. Maniatis, CSH, "Molecular Cloning", 1989). Eine andere Methode beinhaltet die Immobilisierung der Nukleinsäuren an einer festen Phase, z.B. mittels Silika-Adsorptionstechnologie. Diese Methoden haben alle den Nachteil, dass kleinere Nukleinsäuren nicht oder nur in ungenügendem Ausmaß isoliert oder zumindest aufgereinigt werden können.

Zur Lösung dieses Problems sind aus dem Stand der Technik Verfahren zur spezifischen Anreicherung von kleinen RNA-Populationen bekannt, die auf der Silika-Membrantechnologie basieren. Bei diesen Verfahren wird nach der Lyse der Zellen dem Zelllysat eine bestimmte, relativ kleine Menge an Alkohol zugesetzt und zumindest ein Teil der längeren Nukleinsäuren unter chaotropen Bindebedingungen an die Silika-Membran gebunden. Die Alkoholmenge ist in den im Stand der Technik beschriebenen Aufreinigungsverfahren jedoch zu gering, um effizient auch kleine Nukleinsäuren an die Silika-Membran zu binden, so dass diese kleinen RNAs im Durchbruch vorliegen. Im Durchbruch wird nun die Alkoholkonzentration erhöht und der Durchbruch an eine zweite Silika-Membran gebunden. Nach Waschschritten wird die kleine RNA zusammen mit allen anderen Nukleinsäuren, die nicht an die erste Säule gebunden wurden, eluiert (siehe z. B. *mir*Vana®-Kit der Firma Ambion, Austin, USA oder RNeasy® Lipid Tissue Mini Kit der Firma QIAGEN, Hilden, Germany, anzuwenden mittels des *"user developed protocols*")*.*

Sowohl die QIAGEN- als auch die Ambion-Methode haben den Nachteil, dass zwei feste Phasen verwendet werden müssen und es nicht möglich ist, mit Hilfe eines einzigen Bindeschrittes nur die gewünschte kleine RNA zu erhalten. Zudem ist es mit dieser Methode nicht möglich, zum Beispiel ausschließlich miRNAs mit einer Größe von etwa 22 Nukleotiden zu isolieren, ohne noch gleichzeitig tRNAs und weitere größere Nukleinsäuren zu isolieren. Es lässt sich mit dieser Methode unter Umständen zwar die kleine RNA, insbesondere die miRNA, zu einem gewissen Grad anreichern, sie ist jedoch nach wie vor noch mit anderen Nukleinsäuren, insbesondere mit transfer-RNA (tRNA), kontaminiert.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, ein Verfahren zur Anreicherung von kleinen Nukleinsäuren, insbesondere von miRNA, anzugeben, mit dem kleine Nukleinsäuren aus komplexen biologischen Zusammensetzungen, wie beispielsweise aus Zelllysaten, mit möglichst wenigen Verfahrensschritten angereichert werden können.

Auch war es eine Aufgabe der vorliegenden Erfindung, ein Verfahren zur Aufreinigung von kleinen Nukleinsäuren anzugeben, mit dem es möglich ist, insbesondere Nukleinsäuren mit einer Länge von 25 Nukleotiden oder weniger, wie etwa miRNAs, nicht nur von Nukleinsäuren mit einer Länge von mehr als 300 Nukleotiden von anderen Komponenten in einer komplexen biologischen Zusammensetzung, wie beispielsweise einem Zelllysat, abzutrennen, sondern diese kleinen Nukleinsäuren auch von anderen Nukleinsäuren mit einer Länge von weniger als 300 und mehr als 25 Nukleotiden, beispielsweise von tRNAs, gezielt abzutrennen.

Auch sollte ein Verfahren angegeben werden, mit dem möglichst individuell ein gewünschter Größen-Ausschluss für die aufzureinigende RNA generiert werden kann.

Eine weitere Aufgabe der vorliegenden Erfindung ist es, ein Verfahren anzugeben, das ohne Wechsel des Reaktionsgefässes - als sog. "Eintopfreaktion" - durchführbar ist und damit die Verwechslungsgefahr der zu analysierenden Proben auf ein Minimum reduziert.

Weiterhin lag der vorliegenden Erfindung die Aufgabe zugrunde, ein Kit anzugeben, mit dessen Hilfe die vorstehend beschriebene, vorteilhafte Aufreinigung von kleinen Nukleinsäuren, insbesondere von kleiner RNA, aus komplexen biologischen Zusammensetzungen durchgeführt werden kann.

Einen Beitrag zur Lösung der eingangs genannten Aufgaben leistet ein Verfahren zur Anreicherung von Nukleinsäuren mit einer Länge von weniger als 300 Nukleotiden, vorzugsweise weniger als 200 Nukleotiden, besonders bevorzugt weniger als 100 Nukleotiden, darüber hinaus bevorzugt weniger als 50 Nukleotiden und am meisten bevorzugt weniger als 25 Nukleotiden, umfassend die Verfahrensschritte
i) Bereitstellen einer fluiden, vorzugsweise wässrigen, Phase P₁ beinhaltend
   (α1) mindestens eine Nukleinsäure mit einer Länge von weniger als 300 Nukleotiden, vorzugsweise weniger als 200 Nukleotiden, besonders bevorzugt weniger als 100 Nukleotiden, darüber hinaus bevorzugt weniger als 50 Nukleotiden und am meisten bevorzugt weniger als 25 Nukleotiden, sowie
   (α2) mindestens eine von dieser Nukleinsäure (α1) verschiedene Komponente,
ii) in Kontakt bringen der Phase P₁ mit einer Anionenaustausch-Matrix zum Anbinden der Nukleinsäure (α1) an die Anionenaustausch-Matrix,
iii) gegebenenfalls Waschen der Anionenaustausch-Matrix mit einem Waschpuffer, wobei die Nukleinsäure (α1) an der Anionenaustausch-Matrix gebunden bleibt, sowie
iv) Abtrennen, vorzugsweise Eluieren, der an der Anionenaustausch-Matrix gebundenen Nukleinsäure (α1) von der Anionenaustausch-Matrix unter Erhalt einer fluiden, vorzugsweise wässrigen, Phase P₂ beinhaltend die Nukleinsäure (α1).

Völlig überraschend wurde festgestellt, dass sich kleine Nukleinsäuren mit einer Länge von weniger als 300 Nukleotiden aus komplexen biologischen Zusammensetzungen, welche neben diesen kleinen Nukleinsäuren zahlreiche andere Komponenten enthalten können, durch Anbinden an eine Anionenaustausch-Matrix und anschließendes Waschen und Eluieren anreichern lassen, ohne dass es erforderlich ist, zunächst längere Nukleinsäuren abzureichern, wie dies bei den eingangs beschriebenen QIAGEN- und Ambion-Methoden erforderlich ist.

Gemäß einer bevorzugten Ausführungsform des erfindungsgemäßen Verfahrens handelt es sich bei der aufzureinigenden Nukleinsäure (α1) um eine einzelsträngige oder doppelsträngige, vorzugsweise doppelsträngige RNA. Insbesondere ist es bevorzugt, dass es sich bei der RNA mit einer Länge von weniger als 300 Nukleotiden um eine RNA ausgewählt aus der Gruppe beinhaltend miRNA, pre-miRNA, siRNA, snRNA, snoRNA, tRNA, 5S-rRNA, 5,8S-rRNA oder Mischungen aus mindestens zwei davon, insbesondere um eine Mischung aus miRNA und tRNA, handelt, wobei miRNA mit einer Länge in einem Bereich von 15 bis 30 Nukleotiden, darüber hinaus bevorzugt 17 bis 24 Nukleotiden und noch mehr bevorzugt mit einer Länge von 20 bis 23 Nukleotiden als Nukleinsäure (α1) am meisten bevorzugt ist.

Unter den Begriffen "5S-rRNA" bzw. 5,8S-rRNA" werden nicht kodierende Ribonukleinsäuren verstanden, die in eukaryotischen Ribosomen zu finden sind. Unter dem Begriff "tRNA" wird eine Ribonukleinsäure verstanden, die aus etwa 80 Nukleotiden besteht und in der Paarungen konjugierender Basen (Adenin und Uracil; Cytosin und Guanin) auftreten. Diese Paarungen sind die Ursache für die Kleeblattartige Struktur der tRNA. Unter dem Begriff "siRNA" werden Ribonukleinsäuren mit einer Länge von etwa 22 Nukleotiden verstanden, die durch Spaltung einer doppelsträngigen RNA (dsRNA) durch das Enzym "Dicer" entstehen und in den Enzymkomplex "RISC" (RNA-induced silencing complex) eingebaut werden. Unter dem Begriff "snRNA" werden etwa 100 bis 300 Basenpaare große, katalytisch aktive RNAs im Zellkern von Eukaryonten verstanden. Diese snRNAs liegen immer mit Proteinen assoziiert in sogenannten snRNPs (small nuclear ribonucleoproteins) vor und sind verantwortlich für das Splicen der Introns von prä-mRNA zu mRNA. Unter dem Begriff "snoRNA" wird eine Klasse von Ribonukleinsäuren verstanden, die an der chemischen Modifizierung von ribosomaler RNA (rRNA) und andern RNA-Genen, beispielsweise an deren Methylierung, beteiligt sind. Sie bilden einen Bestandteil des sogenannten snoRNPs (small nucleolar ribonucleoprotein). Unter dem Begriff "miRNA" werden kleine Nukleinsäuren verstanden, die zur Regulierung von Entwicklungsprozessen bei Pflanzen und Tieren dienen. Sie binden spezifisch an mRNA und verhindern deren Aktivität in der Translation, so dass z.B. Wachstumsfaktoren nicht in übermäßiger Menge hergestellt werden. miRNAs sind einzelsträngige RNA-Moleküle, die aus einer doppelsträngigen Vorstufe entstehen.

Bei der von den Nukleinsäuren mit einer Länge von höchstens 300 Nukleotiden (α1) verschiedenen Komponente (α2) handelt es sich insbesondere um Nukleinsäuren mit einer Länge von mindestens 300 Nukleotiden (α2') sowie um von Nukleinsäuren verschiedene Komponenten (α2").

Nukleinsäuren mit einer Länge von mindestens 300 Nukleotiden (α2') umfassen insbesondere einzelsträngige oder doppelsträngige DNA- oder einzelsträngige oder doppelsträngige RNA-Moleküle, beispielsweise mRNA, 18S-rRNA oder 28S-rRNA.

Als von den Nukleinsäuren verschiedene Komponenten (α2") kommen insbesondere solche Komponenten in Frage, die bei der Lyse einer Zelle freigesetzt werden. Diese Komponenten umfassen demnach insbesondere Proteine, Lipide, Polypeptide oder Polysaccharide.

Bei der im Verfahrensschritt i) bereitgestellten fluiden, vorzugsweise wässrigen Phase P₁ kann es sich um ein zellfreies Probenmaterial, um ein Plasma, um ein Serum, um eine Körperflüssigkeit, wie beispielsweise Blut, Urin, Sperma, Speichel, Cerebrospinalflüssigkeit, Sputum oder einen Abstrich, um Abwasser, Klärschlamm oder aber um ein Zelllysat, beispielsweise ein Lysat von Zellen aus tierischen oder pflanzlichen Geweben, aus Mikroorganismen wie etwa Bakterien, Pilzen oder Hefen, aus Gewebe- oder Zellkulturen, oder von Zellen aus Körperflüssigkeiten, wie etwa dem Blut, handeln.

Gemäß einer besonderen Ausführungsform des erfindungsgemäßen Verfahrens handelt es sich bei der im Verfahrensschritt i) bereitgestellten fluiden, vorzugsweise wässrigen Phase P₁ um ein Zelllysat, welches erhältlich ist durch ein Verfahren, umfassend die Verfahrensschritte:
I) Bereitstellen von Zellen,
II) Lyse der Zellen unter Erhalt eines Zelllysates, sowie
III) gegebenenfalls mindestens teilweises Abtrennen der mindestens einen, von der Nukleinsäure (α1) verschiedenen Komponente (α2) aus dem Zelllysat.

Bei den Zellen, welche im Verfahrensschritt I) bereitgestellt werden, kann es sich um einen gegebenenfalls fixierten Gewebeschnitt oder ein gegebenenfalls fixiertes Gewebefragment, um kultivierte, adhärente Zellen, um kultivierte Zellen in Suspension oder aber um eine Zelle in einer Körperflüssigkeit handeln.

Sofern es sich bei den Zellen um adhärente Zellen oder um Zellen, welche sich in einem Gewebeverband befinden, handelt, kann der Verfahrensschritt I) gegebenenfalls das Waschen der adhärierten Zellen bzw. des Gewebes, das Ablösen der adhärierten Zellen bzw. das herauslösen der Zellen aus dem Gewebeverband mit geeigneten Enzymlösungen, mit Lösungen beinhaltend komplexierende Verbindungen, wie beispielsweise EDTA, oder Mischungen hieraus, gegebenenfalls das Abtrennen bestimmter Zellpopulationen aus den so erhaltenen Zellsuspensionen, beispielsweise mittels eines Zellsortierers, das Pelletieren der abgelösten bzw. herausgelösten Zellen, das Waschen des so erhaltenen Zellpellets sowie gegebenenfalls das Resuspendieren in einem geeigneten Suspensionspuffer umfassen. Denkbar ist aber auch, die adhärenten Zellen, gegebenenfalls nach einem Waschschritt, ohne vorheriges Ablösen zu lysieren.

Sofern es sich bei den Zellen um kultivierte Zellen in Suspension oder um Zellen in einer Körperflüssigkeit handelt, umfasst der Verfahrensschritt I) vorzugsweise das Pelletieren der suspendierten Zellen, gegebenenfalls nach einem Abtrennen bestimmter Zellpopulationen, beispielsweise mittels eines Zellsortierers, das Waschen des so erhaltenen Zellpellets sowie gegebenenfalls das Resuspendieren in einem geeigneten Suspensionspuffer.

Der Suspensionspuffer, in dem die pelletierten Zellen gegebenenfalls resuspendiert werden, beinhaltet vorzugsweise eine oder mehrere Puffersubstanzen sowie gegebenenfalls eine oder mehrere komplexierende Verbindungen. Der pH-Wert des Suspensionspuffers kann über einen weiten Bereich variiert werden und liegt zur Durchführung des erfindungsgemäßen Verfahrens bevorzugt in einem Bereich von pH 3 bis 11, darüber hinaus bevorzugt in einem Bereich von 5 bis 10 und am meisten bevorzugt in einem Bereich von pH 7 bis 9. Dabei können die dem Fachmann bekannten Puffersysteme zum Einstellen des pH-Werts verwendet werden. Erfindungsgemäß werden bevorzugt auf Tris(hydroxymethyl)-aminomethan (TRIS), Morpholinopropansulfonsäure (MOPS), oder 2-[4-(2-Hydroxyethyl)-1-piperazino]ethansulfonsäure (HEPES) basierende Puffersysteme verwendet, welche die Pufferkomponente in einer Konzentration in einem Bereich von 0,5 bis 100 mMol/l, darüber hinaus bevorzugt in einem Bereich von 1 bis 50 mMol/l und am meisten bevorzugt in einem Bereich von 2,5 bis 25 mMol/l beinhalten. Denkbar sind auch auf Alkaliacetat/Essigsäure basierende Puffersystem oder Mischungen aus einem Alkaliacetat/Essigsäure-Puffersystem und einem Tris(hydroxymethyl)amino-methan-Puffersystem. Als komplexierende Verbindungen können ebenfalls alle Verbindungen eingesetzt werden, die in der Lage sind, insbesondere Kalzium-Ionen zu komplexieren. Eine bevorzugte komplexierende Verbindung ist Ethylendiamintetraacetat (EDTA), welches im Suspensionspuffer vorzugsweise in einer Menge in einem Bereich von 0,01 bis 20 mMol/l, darüber hinaus bevorzugt 0,1 bis 15 mMol/l und am meisten bevorzugt 0,5 bis 5 mMol/l vorliegt.

Die Menge an einzusetzendem Suspensionspuffer ist abhängig von der Zahl der bereitgestellten Zellen. Üblicherweise wird der Suspensionspuffer in einer Menge in einem Bereich von 10 bis 2.000 µl, besonders bevorzugt 50 bis 1.000 µl und am meisten bevorzugt 100 bis 500 µl pro 10⁶ Zellen eingesetzt.

Ein erfindungsgemäß besonders geeigneter Suspensionspuffer ist ein Puffer, welcher 0,5 bis 100 mMol/l, besonders bevorzugt 1 bis 50 mMol/l und am meisten bevorzugt etwa 2,5 bis 25 mMol/l Tris(hydroxymethyl)aminomethan und 0,01 bis 20 mMol/l, besonders bevorzugt 0,1 bis 15 mMol/l und am meisten bevorzugt 0,5 bis 5 mMol/l EDTA beinhaltet und der einen pH-Wert in einem Bereich von 7 bis 9, besonders bevorzugt von etwa 8 aufweist.

Im Verfahrens schritt II) werden die bereitgestellten Zellen lysiert, wobei zur Lyse der Zellen alle dem Fachmann bekannten Lyseverfahren eingesetzt werden können, die geeignet sind, um insbesondere RNA-Material aus Zellen freizusetzen. Als Lyseverfahren kommen insbesondere die Lyse durch Hitzeinwirkung, die Lyse durch mechanische Krafteinwirkung, die Lyse durch Enzyme, wie beispielsweise Proteinkinase K, oder die Lyse durch das in Kontakt bringen der Zellen mit einem Lysepuffer beinhaltend ein Detergenz oder eine chaotrope Verbindung oder mittels hypothonischer Lösungen in Betracht. Gegebenenfalls können die vorstehend genannten Maßnahmen auch miteinander kombiniert werden, indem beispielsweise die Zellen in einem Lysepuffer beinhaltend ein Detergenz oder eine chaotrope Verbindung mechanisch aufgeschlossen werden oder indem beispielsweise ein Lysepuffer beinhaltend Proteinkinase K zusammen mit einer chaotropen Verbindung eingesetzt wird.

Erfindungsgemäß besonders bevorzugt erfolgt die Lyse der Zellen durch einen Lysepuffer beinhaltend ein Detergenz, ein Enzym, eine chaotrope Verbindung oder eine Mischung aus mindestens zwei dieser Komponenten.

Geeignete Detergentien sind aus dem Stand der Technik in großer Zahl bekannt. Erfindungsgemäß besonders bevorzugte Detergentien sind ausgewählt aus der Gruppe beinhaltend Natriumdodecylsulfat (SDS), Polyethylenglykol-phenolether wie beispielsweise Triton-X-100, Tween, NP-40 oder Mischungen hieraus, wobei SDS und Triton-X-100 als Detergenzien besonders bevorzugt sind. Sofern SDS als Detergenz eingesetzt wird, ist es erfindungsgemäß weiterhin bevorzugt, dass pro Mol SDS 1 bis 30 Mol, vorzugsweise 2 bis 20 Mol und am meisten bevorzugt 3 bis 6 Mol NaOH oder KOH, besonders bevorzugt NaOH, zur Lyse der Zellen eingesetzt werden. Sofern der Lysepuffer Detergenzien beinhaltet, ist es weiterhin in dem erfindungsgemäßen Verfahren bevorzugt, dass die Lyse der Zellen im Verfahrensschritt II) in Gegenwart von 0,01 bis 100 µMol, besonders bevorzugt 0,1 bis 50 µMol und am meisten bevorzugt 0,25 bis 5 µMol Detergenz pro 10⁶ Zellen erfolgt. Üblicherweise erfolgt bei der Verwendung eines Detergenz zur Lyse der Zellen die Lyse der Zellen in Gegenwart einer Detergenz-Konzentration von 0,005 bis 5 % (v/v), besonders bevorzugt 0,01 bis 1 % (v/v) und am meisten bevorzugt 0,025 bis 0,5 % (v/v), sofern es sich bei dem Detergenz um eine bei Raumtemperatur und Atmosphärendruck flüssige Verbindungen handelt, oder aber in Gegenwart einer Detergenz-Konzentration von 0,01 bis 1 Gew.-%, besonders bevorzugt 0,25 bis 5 Gew.-% und am meisten bevorzugt 0,05 bis 0,4 Gew.-%, sofern es sich bei dem Detergenz um eine bei Raumtemperatur und Atmosphärendruck feste Verbindungen handelt.

Als chaotrope Verbindung sind insbesondere chaotrope Salze bevorzugt. Unter einem chaotropen Salz wird im Sinne der Erfindung vorzugsweise ein Salz verstanden, das eine hohe Affinität (Bestreben, Anziehung) zu Wasser hat und deshalb eine große feste Hydrathülle (schalenartige Anlagerung von Wassermolekülen) ausbildet. Bevorzugte chaotrope Salze sind insbesondere Guanidiniumisothiocyanat oder Guanidinium-Hydrochlorid, wobei Guanidinium-isothiocyanat besonders bevorzugt ist. Sofern zur Lyse der Zellen chaotrope Salze eingesetzt werden, ist es weiterhin in dem erfindungsgemäßen Verfahren bevorzugt, dass die Lyse der Zellen im Verfahrensschritt II) bei einer Konzentration des chaotropen Salzes von 0,5 bis 10 Mol/l, besonders bevorzugt von 1 bis 5 Mol/l und am meisten bevorzugt von 2 bis 3,5 Mol/l erfolgt. Sofern der Lysepuffer chaotrope Salze beinhaltet, kann es gegebenenfalls auch vorteilhaft sein, wenn der Lysepuffer ein mit Wasser mischbares organisches Lösungsmittel, beispielsweise einen mit Wasser mischbaren Alkohol wie Ethanol oder Isopropanol, in einer Menge in einem Bereich von 10 bis 60 Vol-%, besonders bevorzugt 20 bis 50 Vol-%, beinhaltet.

Als Enzym sind insbesondere Proteasen bevorzugt, wobei unter diesen Trypsin, Proteinase K, Chymotrypsin, Papain, Pepsin, Pronase und Endoproteinase Lys-C. besonders bevorzugt und Proteinase K am meisten bevorzugt ist. Die Konzentration des Enzyms im Lysepuffer liegt vorzugsweise in einem Bereich von 0,01 bis 10 Gew.-%, besonders bevorzugt 0,1 bis 5 Gew.-% und am meisten bevorzugt 0,2 bis 1 Gew.-%, jeweils bezogen auf das Gesamtgewicht des Lysepuffers.

Die Konzentration an Detergenz, an chaotropem Salz oder an Enzym im Lysepuffer ist unter anderem von der Menge der zu lysierenden Zellen sowie von der Art und Weise der Bereitstellung der Zellen im Verfahrensschritt I) abhängig. Werden die zu lysierenden Zellen zunächst in einem Suspensionspuffer suspendiert, so enthält der Lysepuffer das Detergenz, das chaotrope Salz oder das Enzym in einer Konzentration, die höher ist als die Konzentration dieser Komponenten, die während der Lyse der Zellen vorliegen soll. Von diesem konzentrierten Lysepuffer wird dann der Zellsuspension eine solche Menge zugesetzt, die ausreicht, um in der Zellsuspension eine für eine möglichst vollständige Lyse der Zellen erforderliche und vorstehend beschriebene Konzentration an chaotropem Salz, Detergenz bzw. Enzym einzustellen. Wird hingegen der Lysepuffer beispielsweise direkt auf adhärente Zellen appliziert oder mit einem Zellpellet in Kontakt gebracht, so enthält der Lysepuffer das Detergenz, das chaotrope Salz oder das Enzym vorzugsweise in der Konzentration, die auch während der Lyse der Zellen vorliegt.

Gemäß einer besonderen Ausführungsform des erfindungsgemäßen Verfahrens erfolgt die Lyse der Zellen in Gegenwart von 0,1 bis 1 Mol/l, besonders bevorzugt 0,2 bis 0,8 Mol/l und am meisten bevorzugt 0,3 bis 0,7 Mol/l eines Alkalisalzes, wobei Natriumchlorid, Kaliumchlorid und Litiumchlorid bevorzugt sind und Natriumchlorid besonders bevorzugt ist. Werden die zu lysierenden Zellen zunächst in einem Suspensionspuffer suspendiert, so kann entweder bereits dem Suspensionspuffer eine entsprechende Menge des Alkalisalzes zugesetzt werden oder aber es wird ein Lysepuffer dem Suspensionspuffer zugesetzt, der das Alkalisalz in entsprechend höherer Konzentration beinhaltet. Wird hingegen der Lysepuffer beispielsweise direkt auf adhärente Zellen appliziert oder mit einem Zellpellet in Kontakt gebracht, so ist es bevorzugt, dass der Lysepuffer das Alkalisalz in den vorstehend beschriebenen Konzentrationsbereichen beinhaltet.

Ein erfindungsgemäß besonders geeigneter Lysepuffer, welcher einer Zellsuspension zugesetzt werden kann, ist ein Puffer, der 1 bis 200 mMol/l, besonders bevorzugt 5 bis 150 mMol/l und am meisten bevorzugt etwa 10 bis 100 mMol/l NaOH und 0,01 bis 1 % (v/v), besonders bevorzugt 0,025 bis 0,5 % (v/v) und am meisten bevorzugt 0,05 bis 0,4 % (v/v) SDS beinhaltet und der einen pH-Wert in einem Bereich von 5 bis 7, besonders bevorzugt von etwa 5,5 aufweist. Dabei ist es bevorzugt, dass dieser Lysepuffer der Zellsuspension in einem Volumenverhältnis von vorzugsweise 3 : 1 bis 1 : 3, besonders bevorzugt 2 : 1 bis 1 : 2 und am meisten bevorzugt in einem Volumenverhältnis von etwa 1 : 1 zugesetzt wird.

Ein erfindungsgemäß besonders geeigneter Lysepuffer, welcher einem Zellpellet, adhärenten Zellen oder einem Gewebeschnitt- bzw. Gewebefragment zugesetzt werden kann, ist
- ein Puffer, der 0,1 bis 1 Mol/l, besonders bevorzugt 0,25 bis 0,75 Mol/l und am meisten bevorzugt etwa 0,4 bis 0,6 Mol/l NaCl und 0,1 bis 10 % (v/v), besonders bevorzugt 0,5 bis 5 % (v/v) und am meisten bevorzugt 0,75 bis 1,5 % (v/v) Triton-X-100 beinhaltet und der einen pH-Wert in einem Bereich von 6 bis 8, besonders bevorzugt von etwa 7 aufweist,
   oder
- ein Puffer, der 0,5 bis 10 Mol/l, besonders bevorzugt 1 bis 5 Mol/l und am meisten bevorzugt etwa 1,5 bis 3 Mol/l Guanidiniumisothiocyanat, 1 bis 50 mMol/l, besonders bevorzugt 5 bis 40 mMol/l und am meisten bevorzugt 10 bis 20 mMol/l Natriumcitrat sowie 10 bis 60 % (v/v), besonders bevorzugt 20 bis 50 % (v/v) und am meisten bevorzugt 30 bis 40 % (v/v) Ethanol beinhaltet und der einen pH-Wert in einem Bereich von 6 bis 8, besonders bevorzugt von etwa 7 aufweist,
wobei diese Lysepuffer den zu lysierenden Zellen vorzugsweise in einer Menge in einem Bereich von 50 bis 2.000 µl, besonders bevorzugt 100 bis 1.000 µl und am meisten bevorzugt 150 bis 300 µl pro 10⁶ Zellen zugesetzt werden.

Wenn es sich bei den Zellen um einen Gewebeschnitt oder ein Gewebefragment handelt, so umfasst das Bereitstellen der Zellen im Verfahrensschritt I) vorzugsweise das in Kontakt bringen des Gewebeschnittes bzw. des Gewebefragmentes mit flüssigem Stickstoff unmittelbar nach der Entnahme aus einer Pflanze oder einem Tier. Der Gewebeschnitt oder das Gewebefragment wird dann vorzugsweise direkt mit dem Lysepuffer in Kontakt gebracht und gegebenenfalls mittels einer geeigneten Homogenisierungsvorrichtung homogenisiert.

Nachdem die Zellen mit dem Lysepuffer in Kontakt gebracht worden sind, erfolgt die Lyse der Zellen vorzugsweise in einem Temperaturbereich von 15 bis 40°C, besonders bevorzugt jedoch bei Raumtemperatur für eine Zeitdauer in einem Bereich von 1 bis 60 Minuten, besonders bevorzugt 2 bis 15 Minuten.

Bevor nun das Zelllysat als wässrige Phase P₁ im Verfahrensschritt ii) mit der Anionenaustausch-Matrix in Kontakt gebracht wird, kann es gemäß einer besonderen Ausführungsform des erfindungsgemäßen Verfahrens noch vorteilhaft sein, zuvor eine oder mehrere der von der Nukleinsäure (α1) verschiedenen Komponenten (α2) aus dem Zelllysat abzutrennen. Für dieses Abtrennen können grundsätzlich alle dem Fachmann bekannten Trennverfahren, wie beispielweise Fällungsreaktionen, eine Trennung durch Dialyse oder Chromatographie oder aber eine Extraktion, zur Anwendung kommen, wobei eine Extraktion, insbesondere eine Extraktion mit saurem Phenol oder Mischungen aus Phenol und Chloroform besonders bevorzugt und eine Extraktion mit saurem Phenol am meisten bevorzugt ist. Dabei wird das sauere Phenol mit dem Zelllysat vorzugsweise in einem Volumenverhältnis in einem Bereich von 3 : 1 bis 1 : 3, besonders bevorzugt von 2 : 1 bis 1 : 2 und am meisten bevorzugt in einem Volumenverhältnis von etwa 1 : 1 in Kontakt gebracht und gründlich vermischt, beispielsweise mit einem Vortex. Anschließend wird die Zusammensetzung zentrifugiert und die wässrige Phase von der organischen Phase abgetrennt. In der abgetrennten wässrigen Phase, die nunmehr als Phase P₁ dem Verfahrensschritt ii) zugeführt wird, sind auf diese Weise eine oder mehrere von der Nukleinsäure (α1) verschiedene Komponenten abgereichert worden.

Im Verfahrensschritt ii) des erfindungsgemäßen Verfahrens wird nun die wässrige Phase P₁ mit einer Anionenaustausch-Matrix in Kontakt gebracht, um die Nukleinsäure (α1) an die Anionenaustausch-Matrix anzubinden.

Als Anionenaustausch-Matrix können dabei grundsätzlich alle Materialien eingesetzt werden, welche funktionelle Gruppen aufweisen, die bei den Bedingungen, unter denen die wässrige Phase P₁ mit der Anionenaustausch-Matrix in Kontakt gebracht wird, insbesondere unter den pH-Bedingungen, zumindest teilweise in kationischer Form vorliegen.

Bei der Anionenaustausch-Matrix handelt es sich vorzugsweise um einen Feststoff umfassend ein elektrisch neutrales Grundgerüst. Dieses Grundgerüst ist definiert durch die Größe, Form, Porosität, mechanischen Eigenschaften und die positiv geladenen, bevorzugt kovalent an das Feststoffgerüst gebundenen funktionellen Gruppen. Die drei häufigsten Klassen von Grundgerüstmaterialien sind Kieselsäure, Polysaccharide und synthetische Polyolefine, wobei als Polyolefine hauptsächlich Polystyrol- oder Poly(meth)acrylharze zur Anwendung kommen. Poly(meth)acrylharze umfassen Polymere zahlreicher substituierter (Meth)Acrylsäureamide (= Poly(meth)acrylamide) und (Meth)Acrylsäureester (= Poly(meth)-acrylate), wobei das (Meth)Acrylsäuremonomer Alkylsubstituenten am C-2 oder am C-3-Atom tragen kann. Als funktionelle Gruppen, welche an das Grundgerüst gebunden sind, sind funktionelle Gruppen ausgewählt aus der Gruppe beinhaltend primäre, sekundäre oder tertiäre Amino-Gruppen, Phosphin-Gruppen, Hydrazin-Gruppen und Imin-Gruppen besonders bevorzugt. Als Anionenaustausch-Matrix am meisten bevorzugt sind Gerüstmaterialien, an denen Diethylaminoethyl-Gruppen (DEAE, [CH₃CH₂)₂N-CH₂-CH₂-]ₙ) kovalent gebunden sind, insbesondere DEAE-Cellulose, sowie lineare oder verzweigte Polyethylenimine, umfassend [-CH₂-CH₂-NH-]-Gruppen und/oder [-CH₂-CH₂-N(CH₂CH₂NH₂)-]-Gruppen. Weiterhin kann die Anionenaustausch-Matrix als Füllmaterial beispielweise in einer Trennsäule eingesetzt werden. Denkbar ist aber auch, andere Materialien, insbesondere Partikel, Filter, Membranen, Monolithen oder andere organische oder anorganische Oberflächen, wie etwa Mikrotiterplatten, welche nicht aus einem Material mit Anionenaustausch-Eigenschaften bestehen, oder andere Reagiergefäße mit der Anionenaustausch-Matrix zu beschichten.

Erfindungsgemäß besonders bevorzugt ist es dabei, wenn die Anionenaustausch-Matrix in Form einer Beschichtung auf magnetischen oder nichtmagnetischen, ganz besonders bevorzugt jedoch auf magnetischen, am meisten bevorzugt auf superparamagnetischen, ferrimagnitischen oder ferromagentischen Partikeln vorliegt. Magnetische Partikel weisen gegenüber nicht-magentischen Partikeln den Vorteil auf, dass sie magnetische Aggregate bilden und daher schonend, rasch und effizient von der wässrigen Phase P₁ abgetrennt werden können.

Bevorzugte magnetische Partikel, die mit der Anionenaustausch-Matrix beschichtet werden können, sind beispielsweise bei den Firmen Dynal, Advanced Magnetics Inc., Biotechnologies Ltd., Amersham, Promega, Scigen, Advanced Genetic Technologies und Seradyn erhältlich. Geeignete magnetische Partikel sind insbesondere diejenigen, die in der WO-A-83/03920 beschrieben werden sowie die als DYNA-BEADS durch die Firma Dynal AS (Oslo, Norwegen) vertriebenen Partikel. Wenn als Anionenaustausch-Matrix Polyethylenimine eingesetzt werden, so sind Epoxidfunktionalisierte magnetische Partikel, beispielsweise solche, die unter der Bezeichnung "M-PVA E0x" von der Firma Chemagen AG, Baesweiler, Deutschland, erhältlich sind, besonders bevorzugt. Denkbar ist auch der Einsatz Carboxylat-funktionalisierter Partikel, die ebenfalls von der Firma Chemagen AG unter den Produktbezeichnungen "M-PVA C11" oder "M-PVA C12" erhalten werden können. Vorzugsweise weisen die magnetischen Partikel einen mittleren Partikeldurchmesser in einem Bereich von 0,1 bis 100 µm, besonders bevorzugt 0,5 bis 50 µm und am meisten bevorzugt 1 bis 10 µm auf, während die spezifische Oberfläche vorzugsweise in einem Bereich von 0,5 bis 250 m²/g, besonders bevorzugt in einem Bereich von 1 bis 50 m²/g liegt.

Erfindungsgemäß ist es gemäß einer besonderen Ausführungsform des erfindungsgemäßen Verfahrens bevorzugt, dass das Anbinden der Nukleinsäuren (α1) an die Anionenaustausch-Matrix im Verfahrens schritt ii) vorzugsweise bei einem pH-Wert in einem Bereich von 2 bis 10, besonders bevorzugt in einem Bereich von 3 bis 7 und am meisten bevorzugt in einem Bereich von 4 bis 6 erfolgt.

Sofern die im Verfahrensschritt ii) eingesetzte wässrige Phase P₁ einen von diesen pH-Werten abweichenden pH-Wert aufweist, was insbesondere bei der Verwendung eines alkalischen, SDS-haltigen Lysepuffers der Fall sein kann, so kann es notwendig sein, vor oder während des in Kontakt bringens der wässrigen Phase P₁ mit der Anionenaustausch-Matrix den pH-Wert in der wässrigen Phase beispielsweise durch den Zusatz eines Neutralisationspuffers auf den gewünschten Wert einzustellen. Vorzugsweise beinhaltet dieser Neutralisationspuffer ein Alkalisalz der Essigsäure, besonders bevorzugt Kaliumacetat, in einer Konzentration in einem Bereich von 10 bis 10.000 mMol/l, besonders bevorzugt in einem Bereich von 50 bis 5.000 mMol/l und am meisten bevorzugt in einem Bereich von 100 bis 1.000 mMol/l, wobei die Neutralisationslösung vorzugsweise einen pH-Wert in einem Bereich von 2 bis 8, besonders bevorzugt in einem Bereich von 4 bis 6 aufweist. Die Einstellung des pH-Wertes auf einen Wert innerhalb der vorstehend beschriebenen Bereiche in der Lösung des Alkalisalzes der Essigsäure erfolgt vorzugsweise durch den Zusatz von Essigsäure.

Weiterhin kann es gemäß einer besonderen Ausführungsform des erfindungemäßen Verfahrens bevorzugt sein, dass das Anbinden der Nukleinsäure (α1) an die Anionenaustausch-Matrix im Verfahrens schritt ii) in Gegenwart eines Alkalisalzes, vorzugsweise in Gegenwart von Kaliumchlorid, Natriumchlorid oder Litiumchlorid, besonders bevorzugt jedoch in Gegenwart von Natriumchlorid, erfolgt, wobei die Konzentration dieser Alkalisalze beim Anbinden vorzugsweise in einem Bereich von 0,01 bis 10 Mol/l, besonders bevorzugt in einem Bereich von 0,05 bis 5 Mol/l und am meisten bevorzugt in einem Bereich von 0,25 bis 0,75 Mol/l liegt. Die Einstellung dieser Salzkonzentrationen während des Anbindens kann entweder dadurch erfolgen, dass in der bereits vorliegenden wässrigen Phase P₁, beispielsweise einem Zelllysat, eine entsprechend konzentrierte Salzlösung zugesetzt wird oder aber, indem Zellen in Gegenwart eines Suspensionspuffers suspendiert oder aber in Gegenwart eines Lysepuffers lysiert werden, welcher eine entsprechende Salzkonzentration aufweist.

Weiterhin kann es gemäß einer anderen, besonderen Ausführungsform des erfindungemäßen Verfahrens bevorzugt sein, dass das Anbinden der Nukleinsäure (α1) an die Anionenaustausch-Matrix im Verfahrensschritt ii) in Gegenwart einer chaotropen Substanz, insbesondere eines chaotropen Salzes wie etwa Guanidiniumisothiocyanat erfolgt, wobei die Konzentration dieser chaotropen Salze beim Anbinden vorzugsweise in einem Bereich von 0,1 bis 10 Mol/l, besonders bevorzugt in einem Bereich von 0,5 bis 5 Mol/l und am meisten bevorzugt in einem Bereich von 1 bis 3 Mol/l liegt. Weiterhin kann es bei dieser besonderen Ausführungsform des erfindungsgemäßen Verfahrens auch vorteilhaft sein, das Anbinden in Gegenwart von mit Wasser mischbaren, organischen Lösungsmitteln, insbesondere in Gegenwart von Alkholen wie Ethanol oder Isopropanol in einer Konzentration in einem Bereich von 10 bis 70 Vol-%, besonders bevorzugt 20 bis 60 Vol-% durchzuführen.

Das Anbinden der Nukleinsäure (α1) an die Anionenaustausch-Matrix im Verfahrensschritt ii) erfolgt vorzugsweise dadurch, dass im Falle eines Einsatzes der Anionenaustausch-Matrix als Füllmaterial in einer Säule die wässrige Phase P₁, beispielsweise das Zelllysat, in welchem die vorstehend beschriebenen vorteilhaften pH-Bedingungen erfüllt und in dem die vorstehend beschriebenen Salze in den genannten Konzentrationen enthalten sind, über das Säulenmaterial geleitet wird, vorzugsweise bei Temperaturen in einem Bereich von 1 bis 30°C, besonders bevorzugt 2 bis 25°C, beispielsweise bei Raumtemperatur. Das Durchleiten der wässrigen Phase P₁ durch das Säulenmaterial kann gegebenenfalls mittels Überdruck, Vakuum, Zentrifugation oder durch Unterstützung mittels Kapillarkräften erfolgen.

Im Falle eines Einsatzes von mit der Anionenaustausch-Matrix beschichteten Partikeln erfolgt das Anbinden vorzugsweise dadurch, dass die mit den Partikeln in Kontakt gebrachte wässrige Phase P₁ kontinuierlich bewegt wird, beispielsweise mittels eines Schüttlers, wobei auch in diesem Fall das Anbinden vorzugsweise bei Temperaturen in einem Bereich von 1 bis 30°C, besonders bevorzugt 2 bis 25°C, beispielsweise bei Raumtemperatur erfolgt.

Nach dem Anbinden der Nukleinsäuren (α1) an die Anionenaustausch-Matrix kann diese in einem Verfahrensschritt iii) gegebenenfalls mittels eines Waschpuffers gewaschen werden. Wurde die Anionenaustausch-Matrix als Füllmaterial in einer Säule eingesetzt, so erfolgt das Waschen vorzugsweise dadurch, dass der Waschpuffer über die Säule geleitet wird, wobei auch hier ein Überdruck, ein Vakuum, eine Zentrifugation oder Kapillarkräfte genutzt werden können. Wurden beispielsweise mit einer Anionenaustausch-Matrix beschichtete, nicht-magnetische Partikel eingesetzt, so werden diese zunächst von der wässrigen Phase P₁, beispielsweise mittels Filtration oder Zentrifugation, abgetrennt und anschließend mit dem Waschpuffer gewaschen. Bei einem Einsatz von mit einer Anionenaustausch-Matrix beschichteten magnetischen Partikeln erfolgt das Waschen vorzugsweise dadurch, dass das Reaktionsgefäß, in dem sich die mit der wässrigen Phase P₁ in Kontakt gebrachten magnetischen Partikel befinden, mit einem Magneten in Kontakt gebracht wird, so dass aufgrund des Magnetfeldes die magnetischen Partikel an den Innenwänden des Reaktionsgefäßes haften bleiben. Die wässrige Phase P₁ kann unter diesen Umständen leicht abgenommen und durch den Waschpuffer ersetzt werden. Hierfür geeignete Vorrichtungen sind beispielsweise von der Firma Dynal, Oslo, Norwegen, erhältlich.

Bei dem Waschpuffer kann es sich beispielsweise um RNase freies Wasser, um Mischungen aus Wasser und wasserlöslichen organischen Lösungsmitteln, wie etwa Mischungen aus Wasser mit 1 bis 80 Vol-% eines wasserlöslichen Alkohols, beispielsweise mit 1 bis 80 Vol-% Ethanol oder Isopropanol, oder um wässrige Salzlösungen, insbesondere wässrige Acetat-Lösungen, beispielsweise wässrige Natriumacetat-Lösung mit einer Natriumacetat-Konzentration in einem Bereich von 1 bis 50 mMol/l, besonders bevorzugt 5 bis 25 mMol/l, handeln, wobei der Waschpuffer vorzugsweise einen pH-Wert in einem Bereich von 4 bis 9, besonders bevorzugt.

Der Schritt des Waschens kann, je nach Bedarf, ein-, zwei-, dreimal, gegebenenfalls auch noch öfter, unter Einsatz eines jeweils frischen Waschpuffers, wiederholt werden.

Nach dem Anbinden der Nukleinsäure (α1) an die Anionenaustausch-Matrix im Verfahrensschritt ii) und gegebenenfalls dem Waschen im Verfahrensschritt iii) erfolgt im Verfahrensschritt iv) das Abtrennen der an der Anionenaustausch-Matrix gebundenen Nukleinsäure (α1) von der Anionenaustausch-Matrix unter Erhalt einer fluiden, vorzugsweise wässrigen, Phase P₂ beinhaltend die Nukleinsäure (α1).

Vorzugsweise erfolgt dieses Abtrennen durch Eluieren, indem die Anionenaustausch-Matrix mit einem Elutionspuffer in Kontakt gebracht wird, der die Bindung zwischen den funktionellen Gruppen der Anionenaustausch-Matrix und der Nukleinsäure (α1) löst, so dass ein die Nukleinsäure (α1) beinhaltendes Eluat als fluide Phase P₂ erhalten wird.

Wurde die Anionenaustausch-Matrix als Füllmaterial in einer Säule eingesetzt, so erfolgt das Eluieren vorzugsweise dadurch, dass der Elutionspuffer über die Säule geleitet wird, wobei wiederum ein Überdruck, ein Vakuum, eine Zentrifugation oder Kapillarkräfte genutzt werden können. Wurden beispielsweise mit einer Anionenaustausch-Matrix beschichtete, nicht-magnetische Partikel eingesetzt, so werden diese zunächst von der wässrigen Phase P₂ bzw. vom Waschpuffer, beispielsweise mittels Filtration oder Zentrifugation, abgetrennt und anschließend mit dem Elutionspuffer in Kontakt gebracht. Bei einem Einsatz von mit einer Anionenaustausch-Matrix beschichteten magnetischen Partikeln erfolgt das Eluieren vorzugsweise dadurch, dass das Reaktionsgefäß, in dem sich die mit der wässrigen Phase P₁ oder dem Waschpuffer in Kontakt gebrachten magnetischen Partikel befinden, mit einem Magneten in Kontakt gebracht wird, so dass aufgrund des Magnetfeldes die magnetischen Partikel an den Innenwänden des Reaktionsgefäßes haften bleiben. Die wässrige Phase P₁ oder aber der Waschpuffer können unter diesen Umständen leicht abgenommen und durch den Elutionspuffer ersetzt werden.

Bei dem Elutionspuffer handelt es sich vorzugsweise um eine wässrige Salzlösung, insbesondere um wässrige Lösungen beinhaltend Alkalihalogenide, wie etwa NaCl, KCl oder LiCl, Erdalkalihalogenide, wie etwa CaCl₂ oder MgCl₂, Ammoniumsalze wie etwa Ammoniumchlorid oder Ammoniumsulfat oder Mischungen aus mindestens zwei dieser Salze, wobei der Elutionspuffer gegebenenfalls auch Puffersysteme wie etwa Alkaliacetat/Essigsäure oder auf Tris(hydroxymethyl)amino-methan basierende Puffersysteme beinhalten kann.

Gemäß einer besonderen Ausführungsform des erfindungsgemäßen Verfahrens beinhaltet der Elutionspuffer wasserlösliche Kalziumsalze wie CaCl₂, wasserlösliche Magnesiumsalze wie MgCl₂, wasserlösliche Ammoniumsalze wie Ammoniumsulfat oder Ammoniumchlorid oder Mischungen aus mindestens zwei dieser Salze. Wenn der Elutionspuffer CaCl₂ beinhaltet, so liegt dieses Salz vorzugsweise in einer Konzentration in einem Bereich von 1 bis 1.000 mMol/l, besonders bevorzugt 5 bis 500 mMol/l und am meisten bevorzugt 10 bis 100 mMol/l vor. Wenn der Elutionspuffer MgCl₂ beinhaltet, so liegt dieses Salz vorzugsweise in einer Konzentration in einem Bereich von 1 bis 1.000 mMol/l, besonders bevorzugt 5 bis 500 mMol/l und am meisten bevorzugt 10 bis 100 mMol/l vor. Wenn der Elutionspuffer Ammoniumsulfat und/oder Ammoniumchlorid beinhaltet, so liegen diese Salze vorzugsweise in einer Gesamtkonzentration in einem Bereich von 1 bis 1.000 mMol/l, besonders bevorzugt 5 bis 500 mMol/l und am meisten bevorzugt 20 bis 300 mMol/l vor. Der pH-Wert des Elutionspuffers liegt vorzugsweise in einem Bereich von 5 bis 12, vorzugsweise 6 bis 10 und besonders bevorzugt 7 bis 10.

Insbesondere Elutionspuffer, welche als Salze vorzugsweise ausschließlich Kalziumsalze, insbesondere CaCl₂, und/oder Ammoniumsalze, vorzugsweise Ammoniumsulfat und/oder Ammoniumchlorid beinhalten, sind besonders geeignet, um selektiv miRNA im Vergleich zu tRNA anzureichern. So lassen sich mit Elutionspuffern bestehend aus Wasser und CaCl₂ in einer Konzentration von vorzugsweise bis zu 60 mMol/l und auch mit Elutionspuffern bestehend aus Wasser und Ammoniumsulfat oder Ammoniumchlorid in einer Konzentration von vorzugsweise bis zu 170 bis 200 mMol/l eine gute Anreicherung von miRNA bei gleichzeitiger Abreicherung von tRNA erzielen, so dass diese Elutionspuffer insbesondere zur selektiven Anreicherung von miRNA aus miRNA- und tRNA-enthaltenden Zusammensetzungen geeignet sind.

Erfindungsgemäß besonders geeignete Elutionspuffer sind
- ein Elutionspuffer EP₁, der 1 bis 10.000 mMol/l, besonders bevorzugt 10 bis 5.000 mMol/l und am meisten bevorzugt 50 bis 1.000 mMol/l TRIS, 1 bis 1.000 mMol/l, vorzugsweise 5 bis 800 mMol/l und am meisten bevorzugt 10 bis 500 mMol/l eines Alkalisalzes, vorzugsweise NaCl oder KCl, 1 bis 400 mMol/l, besonders bevorzugt 10 bis 300 mMol/l und am meisten bevorzugt 50 bis 200 mMol/ eines Ammoniumsalzes, vorzugsweise Ammoniumsulfat oder Ammoniumchlorid sowie 0,1 bis 200 mMol/l, besonders bevorzugt 0,5 bis 100 mMol/l und am meisten bevorzugt 1 bis 50 mMol/l eines Magnesiumsalzes, vorzugsweise Magnesiumchlorid, gelöst in Wasser, enthält und der vorzugsweise einen pH-Wert in einem Bereich von 7 bis 11, besonders bevorzugt 8 bis 10 aufweist;
- ein Elutionspuffer EP₂, der 1 bis 1.000 mMol/l, besonders bevorzugt 5 bis 500 mMol/l und am meisten bevorzugt 10 bis 100 mMol/l eines Magnesiumsalzes, vorzugsweise Magnesiumchlorid, gelöst in Wasser, enthält und der vorzugsweise einen pH-Wert in einem Bereich von 6 bis 10, besonders bevorzugt 7 bis 9 aufweist;
- ein Elutionspuffer EP₃, der 1 bis 1.000 mMol/l, besonders bevorzugt 5 bis 500 mMol/l und am meisten bevorzugt 10 bis 100 mMol/l eines Kalziumsalzes, vorzugsweise Kalziumchlorid, gelöst in Wasser, enthält und der vorzugsweise einen pH-Wert in einem Bereich von 6 bis 10, besonders bevorzugt 7 bis 9 aufweist;
- ein Elutionspuffer EP₄, der 1 bis 1.000 mMol/l, besonders bevorzugt 5 bis 500 mMol/l und am meisten bevorzugt 20 bis 300 mMol/l eines Ammoniumsalzes, vorzugsweise Ammoniumchlorid oder Ammoniumsulfat, gelöst in Wasser, enthält und der vorzugsweise einen pH-Wert in einem Bereich von 6 bis 10, besonders bevorzugt 7 bis 9 aufweist;
- ein Elutionspuffer EP₅, der 1 bis 2.000 mMol/l, besonders bevorzugt 10 bis 1.000 mMol/l und am meisten bevorzugt 100 bis 500 mMol/l eines Alkalisalzes, vorzugsweise Kaliumchlorid, Natriumchlorid oder Litiumchlorid, , gelöst in Wasser, enthält und der vorzugsweise einen pH-Wert in einem Bereich von 6 bis 10, besonders bevorzugt 7 bis 9 aufweist.

Insbesondere die Elutionspuffer EP₁ bis EP₄ sind geeignet, um miRNA aus miRNA und tRNA enthaltenden Zusammensetzungen aufzureinigen, während der Elutionspuffer EP₅ besonders geeignet ist, um allgemein Nukleinsäuren mit einer Länge von weniger als 300 Nukleotiden, insbesondere weniger als 100 Nukleotiden, aus Zusammensetzungen aufzureinigen, die neben diesen Nukleotiden noch längerkettige Nukleinsäuren beinhalten. Mit steigender Mg²⁺-, Ca²⁺- bzw. NH₄⁺-Konzentration in den Elutionspuffern EP₂ bis EP₄ kann zudem selektiv die Anreicherung von miRNA gegenüber tRNA aus miRNA und tRNA beinhaltenden Zusammensetzungen reguliert werden.

Gemäß einer besonderen Ausführungsform des erfindungsgemäßen Verfahrens ist es bevorzugt, dass die relative Menge an RNA mit einer Länge von weniger als 300 Nukleotiden, vorzugsweise weniger als 100 Nukleotiden und am meisten bevorzugt weniger als 25 Nukleotiden, in der wässrigen Phase P₂, bezogen auf die Gesamtmenge an RNA in der wässrigen Phase P₂, um einen Faktor von mindestens 2, besonders bevorzugt mindestens 4, darüber hinaus bevorzugt mindestens 6, darüber hinaus noch mehr bevorzugt mindestens 10 und am meisten bevorzugt mindestens 20 größer ist als die relative Menge an RNA mit einer Länge von weniger als 300 Nukleotiden, vorzugsweise weniger als 100 Nukleotiden und am meisten bevorzugt weniger als 25 Nukleotiden, in der wässrigen Phase P₁, bezogen auf die Gesamtmenge an RNA in der wässrigen Phase P₁.

Bei einer weiteren besonderen Ausführungsform des erfindungsgemäßen Verfahrens, insbesondere bei derjenigen Ausführungsform, bei der einer der Elutionspuffer EP₁ bis EP₄ eingesetzt wird, ist es bevorzugt, dass die relative Menge an miRNA in der wässrigen Phase P₂, bezogen auf die Gesamtmenge an miRNA und tRNA in der wässrigen Phase P₂, um einen Faktor von mindestens 2, besonders bevorzugt mindestens 4, darüber hinaus bevorzugt mindestens 6, darüber hinaus noch mehr bevorzugt mindestens 10 und am meisten bevorzugt mindestens 20 größer ist als die relative Menge an miRNA in der wässrigen Phase P₁, bezogen auf die Gesamtmenge an miRNA und tRNA in der wässrigen Phase P₁.

Einen Beitrag zur Lösung der eingangs genannten Aufgaben leistet auch ein Kit zur Anreicherung von Nukleinsäuren mit einer Länge von weniger als 300 Nukleotiden, vorzugsweise weniger als 100 Nukleotiden und am meisten bevorzugt weniger als 25 Nukleotiden, umfassend:
(β1) einen Lysepuffer oder ein Lysepufferkonzentrat,
(β2) eine Anionenaustausch-Matrix,
(β3) einen Elutionspuffer,
(β4) gegebenenfalls einen Suspensionspuffer,
(β5) gegebenenfalls einen Neutralisationspuffer,
(β6) gegebenenfalls einen Waschpuffer sowie
(β7) gegebenenfalls eine Extraktionssubstanz, beispielsweise Phenol, ein Alkohol, wie beispielsweise Ethanol, oder Mischungen hieraus.

Mit einem solchen Kit kann das vorstehend beschriebene Verfahren durchgeführt werden.

Als Suspensionspuffer (β4), Lysepuffer (β1), Neutralisationspuffer (β5), Waschpuffer (β6) und Elutionspuffer (β3) sind diejenigen Puffer bevorzugt, die bereits im Zusammenhang mit dem erfindungsgemäßen Verfahren als bevorzugte Puffer genannt worden sind. Bei dem Lysepufferkonzentrat handelt es sich um einen Puffer, welcher die für die Lyse effektive Verbindung, insbesondere das Detergenz oder das chaotrope Salz, in einer Konzentration enthält, die höher ist als die bei der Lyse der Zellen vorliegende Konzentration. Ein solches Lysepufferkonzentrat wird insbesondere dann eingesetzt, wenn als Ausgangsmaterial, aus dem die kurzkettigen Nukleinsäuren isoliert werden sollen, Zellsupensionen eingesetzt werden sollen, in denen dann durch Zugabe definierter Mengen des Lysepufferkonzentrates die für die Lyse erforderlichen Lysebedingungen eingestellt werden können.

Als Anionenaustausch-Matrix (β2) kommen ebenfalls als Materialien in Betracht, die bereits im Zusammenhang mit dem erfindungsgemäßen Verfahren zur Anreicherung von Nukleinsäuren als bevorzugte Anionenaustausch-Matrix genannt wurden, so zum Beispiel insbesondere auch mit einer Anionenaustausch-Matrix beschichtete magnetische oder nicht-magnetische Partikel.

Gemäß einer besonderen Ausführungsform des erfindungsgemäßen Kit beinhaltet dieser als Anionenaustausch-Matrix (β2) mit einer Anionenaustausch-Matrix beschichtete, magnetische Partikel und, als Elutionspuffer (β3) einen der Puffer ausgewählt aus der Gruppe beinhaltend EP₁, EP₂, EP₃ und EP₄.

Einen Beitrag zur Lösung der eingangs genannten Aufgaben leistet weiterhin die Verwendung des vorstehend beschriebenen Kits in dem eingangs beschriebenen, erfindungsgemäßen Verfahren zur Aufreinigung von Nukleinsäuren mit einer Länge von weniger als 300 Nukleotiden, vorzugsweise weniger als 200 Nukleotiden, besonders bevorzugt weniger als 100 Nukleotiden, darüber hinaus bevorzugt weniger als 50 Nukleotiden und am meisten bevorzugt weniger als 25 Nukleotiden.

Einen Beitrag zur Lösung der eingangs genannten Aufgaben leistet auch die Verwendung einer Anionenaustausch-Matrix zur Aufreinigung von Nukleinsäuren mit einer Länge von weniger als 300 Nukleotiden, vorzugsweise weniger als 200 Nukleotiden, besonders bevorzugt weniger als 100 Nukleotiden, darüber hinaus bevorzugt weniger als 50 Nukleotiden und am meisten bevorzugt weniger als 25 Nukleotiden, wobei als Anionenaustausch-Matrix und als Nukleotide diejenigen Verbindungen bevorzugt sind, die bereits eingangs im Zusammenhang mit der erfindungsgemäßen Verfahren zur Aufreinigung von Nukleinsäuren als bevorzugte Komponenten genannt wurden.

Schließlich leistet auch ein Verfahren zur Behandlung einer Krankheit einen Beitrag zu Lösung der eingangs genannten Aufgaben, umfassend die Verfahrensschritte:
(γ1) Diagnose der Krankheit durch ein Diagnoseverfahren, welches die Anreicherung von Nukleinsäuren mit einer Länge von weniger als 300 Nukleotiden, vorzugsweise weniger als 200 Nukleotiden, besonders bevorzugt weniger als 100 Nukleotiden, darüber hinaus bevorzugt weniger als 50 Nukleotiden und am meisten bevorzugt weniger als 25 Nukleotiden, gemäß dem eingangs beschriebenen Aufreinigungsverfahren umfasst, sowie
(γ2) therapeutische Behandlung der diagnostizierten Krankheit.

Als zu behandelnde Krankheit kommen alle Krankheiten in Betracht, deren Ursache oder Verlauf in irgendeiner Weise mit der Art und Menge der in bestimmten Körperzellen oder Körperflüssigkeiten vorliegenden Nukleinsäuren mit einer Länge von weniger als 300 Nukleotiden, vorzugsweise weniger als 200 Nukleotiden, besonders bevorzugt weniger als 100 Nukleotiden, darüber hinaus bevorzugt weniger als 50 Nukleotiden und am meisten bevorzugt weniger als 25 Nukleotiden, insbesondere jedoch mit der Art und Menge der miRNA korreliert, sei es, dass eine Veränderung der Art und Menge dieser Nukleinsäuren, im Vergleich zu einem gesunden Patienten, ursächlich ist für die Krankheit oder dass eine Veränderung der Art und Menge dieser Nukleinsäuren, im Vergleich zu einem gesunden Patienten, eine Folge der Krankheit ist.

Die Erfindung wird nun anhand nicht limitierender Figuren und Beispiele näher erläutert.

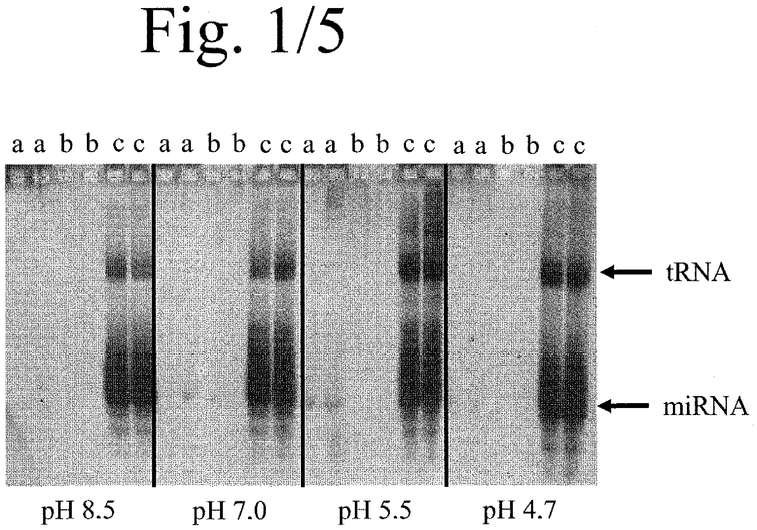

Es zeigt die Figur 1 ein mit Silbernitrat gefärbtes, 15 %iges% Polyacrylamid-Gel, mit dem die im Beispiel 1 erhaltenen Eluate aufgetrennt wurde (Doppelauftragung im Gel; a = Waschpuffer nach dem ersten Waschen, b = Waschpuffer nach dem zweiten Waschen, c = Eluat).

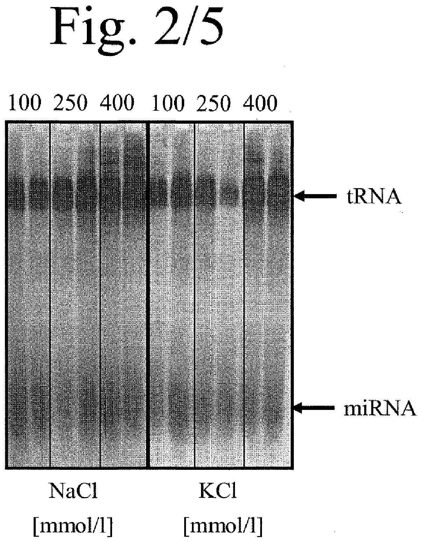

Es zeigt die Figur 2 ein mit Silbernitrat gefärbtes, 15 %iges% Polyacrylamid-Gel, mit dem die im Beispiel 2 erhaltenen Eluate aufgetrennt wurde (Doppelauftragung im Gel).

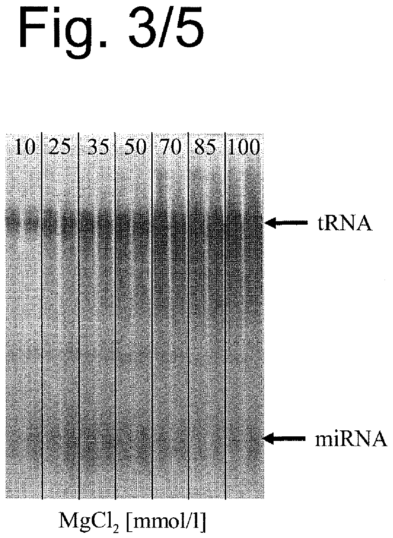

Es zeigt die Figur 3 ein mit Silbernitrat gefärbtes, 15 %iges% Polyacrylamid-Gel, mit dem das im Beispiel 3 erhaltene Eluat aufgetrennt wurde (Doppelauftragung im Gel).

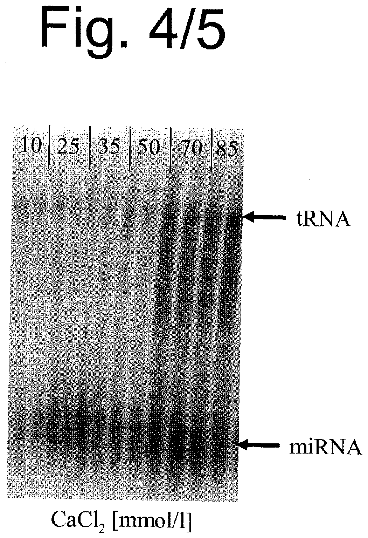

Es zeigt die Figur 4 ein mit Silbernitrat gefärbtes, 15 %iges% Polyacrylamid-Gel, mit dem das im Beispiel 4 erhaltene Eluat aufgetrennt wurde (Doppelauftragung im Gel).

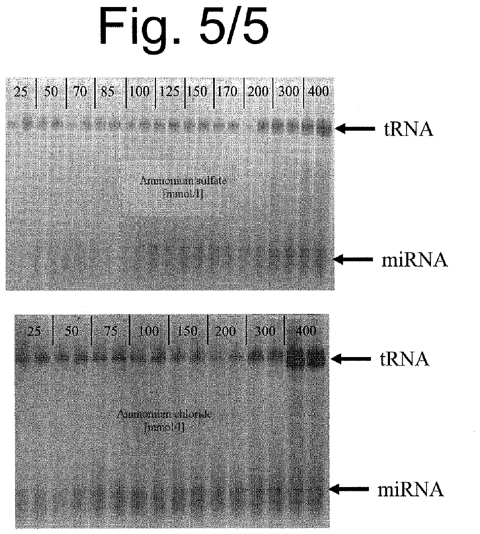

Es zeigt die Figur 5 ein mit Silbernitrat gefärbtes, 15 %iges% Polyacrylamid-Gel, mit dem das im Beispiel 5 erhaltene Eluat aufgetrennt wurde (Doppelauftragung im Gel).

Offenbart werden die folgenden Gegenstände:
1. Ein Verfahren zur Anreicherung von Nukleinsäuren mit einer Länge von weniger als 300 Nukleotiden, umfassend die Verfahrensschritte
   i) Bereitstellen einer fluiden, vorzugsweise wässrigen, Phase P₁ beinhaltend
      (α1) mindestens eine Nukleinsäure mit einer Länge von weniger als 300 Nukleotiden, sowie
      (α2) mindestens eine von dieser Nukleinsäure (α1) verschiedene Komponente,
   ii) in Kontakt bringen der Phase P₁ mit einer Anionenaustausch-Matrix zum Anbinden der Nukleinsäure (α1) an die Anionenaustausch-Matrix,
   iii) gegebenenfalls Waschen der Anionenaustausch-Matrix mit einem Waschpuffer, wobei die Nukleinsäure (α1) an der Anionenaustausch-Matrix gebunden bleibt, sowie
   iv) Abtrennen, vorzugsweise Eluieren, der an der Anionenaustausch-Matrix gebundenen Nukleinsäure (α1) von der Anionenaustausch-Matrix unter Erhalt einer fluiden, vorzugsweise wässrigen, Phase P₂ beinhaltend die Nukleinsäure (α1).
2. Verfahren nach Gegenstand 1, wobei es sich bei der Nukleinsäure (α1) um eine RNA handelt.
3. Verfahren nach Gegenstand 2, wobei die RNA ausgewählt ist aus der Gruppe enthaltend miRNA, pre-miRNA, siRNA, snRNA, snoRNA, tRNA, 5S-rRNA, 5,8S-rRNA oder Mischungen aus mindestens zwei davon.
4. Verfahren nach Gegenstand 3, wobei es sich bei der RNA um miRNA, pre-miRNA, tRNA oder Mischungen aus miRNA und tRNA handelt.
5. Verfahren nach einem der vorhergehenden Gegenstände, wobei die Anionenaustausch-Matrix funktionelle Gruppen ausgewählt aus der Gruppe enthaltend Amino-Gruppen, Phosphin-Gruppen, Hydrazin-Gruppen und Imin-Gruppen aufweist.
6. Verfahren nach einem der vorhergehenden Gegenstände, wobei die Anionenaustausch-Matrix in Form einer Beschichtung auf einem Partikel, einem Filter, einer Membran, einem Monolithen, einer Mikrotiterplatte oder anderen Reagiergefäßen vorliegt.
7. Verfahren nach Gegenstand 6, wobei der Partikel ein magnetischer, vorzugsweise superparamagnetischer, ferrimagnetischer oder ferromagnetischer Partikel ist.
8. Verfahren nach einem der vorhergehenden Gegenstände, wobei das Anbinden der Nukleinsäure (α1) an die Anionenaustausch-Matrix im Verfahrensschritt ii) in Gegenwart von 0,01 bis 10 Mol/l NaCl erfolgt.
9. Verfahren nach einem der vorhergehenden Gegenstände, wobei es sich bei dem Waschpuffer um RNase freies Wasser handelt.
10. Verfahren nach einem der vorhergehenden Gegenstände, wobei das Trennen im Verfahrensschritt iv) durch einen Elutionspuffer erfolgt.
11. Verfahren nach Gegenstand 10, wobei der Elutionspuffer wasserlösliche Kalziumsalze, wasserlösliche Magnesiumsalze, wasserlösliche Ammoniumsalze oder Mischungen hieraus beinhaltet.
12. Verfahren nach Gegenstand 10 oder 11, wobei der Elutionspuffer Kalziumchlorid in einer Konzentration in einem Bereich von 1 bis 1.000 mMol/l beinhaltet.
13. Verfahren nach einem der Gegenstände 10 bis 12, wobei der Elutionspuffer Magnesiumchlorid in einer Konzentration in einem Bereich von 1 bis 1.000 mMol/l beinhaltet.
14. Verfahren nach einem der Gegenstände 10 bis 13, wobei der Elutionspuffer Ammoniumsulfat oder Ammoniumchlorid in einer Konzentration in einem Bereich von 1 bis 1.000 mMol/l beinhaltet.
15. Verfahren nach einem der vorhergehenden Gegenstände, wobei die im Verfahrensschritt i) bereitgestellte, wässrige Phase P₁ ein Zelllysat ist.
16. Verfahren nach Gegenstand 15, wobei das Zelllysat erhältlich ist durch ein Verfahren umfassend die Verfahrensschritte:
   I) Bereitstellen von Zellen,
   II) Lyse der Zellen unter Erhalt eines Zelllysates, sowie
   III) gegebenenfalls mindestens teilweises Abtrennen der mindestens einen, von der Nukleinsäure (α1) verschiedenen Komponente (α2) aus dem Zelllysat.
17. Verfahren nach einem der Gegenstände 2 bis 16, wobei die relative Menge an RNA mit einer Länge von weniger als 300 Nukleotiden in der Phase P₂, bezogen auf die Gesamtmenge an RNA in der Phase P₂, um einen Faktor von mindestens 2 größer ist als die relative Menge an RNA mit einer Länge von weniger als 300 Nukleotiden in der Phase P₁, bezogen auf die Gesamtmenge an RNA in der Phase P₁.
18. Verfahren nach einem der Gegenstände 4 bis 17, die relative Menge an miRNA in der wässrigen Phase P₂, bezogen auf die Gesamtmenge an miRNA und tRNA in der wässrigen Phase P₂, um einen Faktor von mindestens 2 größer ist als die relative Menge an miRNA in der wässrigen Phase P₁, bezogen auf die Gesamtmenge an miRNA und tRNA in der wässrigen Phase P₁.
19. Ein Kit zur Anreicherung von Nukleinsäuren mit einer Länge von weniger als 300 Nukleotiden, umfassend:
   (β1) einen Lysepuffer oder ein Lysepufferkonzentrat,
   (β2) eine Anionenaustausch-Matrix,
   (β3) einen Elutionspuffer,
   (β4) gegebenenfalls einen Suspensionspuffer,
   (β5) gegebenenfalls einen Neutralisationspuffer,
   (β6) gegebenenfalls einen Waschpuffer sowie
   (β7) gegebenenfalls eine Extraktionssubstanz.
20. Kit nach Gegenstand 19, wobei die Anionenaustausch-Matrix (β2) funktionelle Gruppen ausgewählt aus der Gruppe beinhaltend Amino-Gruppen, Phosphin-Gruppen, Hydrazin-Gruppen und Imin-Gruppen aufweist.
21. Kit nach Gegenstand 19 oder 20, wobei die Anionenaustausch-Matrix (β2) in Form einer Beschichtung auf einem Partikeln, einem Filter, einer Membran, einem Monolithen oder einer Mikrotiterplatte vorliegt.
22. Kit nach Gegenstand 21, wobei die Anionenaustausch-Matrix (β2) in Form einer Beschichtung auf einem magnetischen, vorzugsweise einem superparamagnetischen, ferrimagnetischen oder ferromagnetischen Partikel vorliegt.
23. Kit nach einem der Gegenstände 19 bis 22, wobei der Elutionspuffer (β3) Kalziumchlorid in einer Konzentration in einem Bereich von 1 bis 1.000 mMol/l beinhaltet.
24. Kit nach einem der Gegenstände 19 bis 22, wobei der Elutionspuffer (β3) Magnesiumchlorid in einer Konzentration in einem Bereich von 1 bis 1.000 mMol/l beinhaltet.
25. Kit nach einem der Gegenstände 19 bis 22, wobei der Elutionspuffer (β3) Ammoniumsulfat oder Ammoniumchlorid in einer Konzentration in einem Bereich von 1 bis 1.000 mMol/l beinhaltet.
26. Verwendung des Kits nach einem der Gegenstände 19 bis 25 zur Anreicherung von Nukleinsäuren mit einer Länge von weniger als 300 Nukleotiden.
27. Verwendung des Kits nach einem der Gegenstände 19 bis 25 in einem Verfahren nach einem der Gegenstände 1 bis 18.
28. Verwendung einer Anionenaustausch-Matrix zur Anreicherung von Nukleinsäuren mit einer Länge von weniger als 300 Nukleotiden.
29. Ein Verfahren zur Behandlung einer Krankheit, umfassend die Verfahrensschritte:
   (γ1) Diagnose der Krankheit durch ein Diagnoseverfahren, welches die Anreicherung von Nukleinsäuren mit einer Länge von weniger als 300 Nukleotiden gemäß einem Verfahren nach einem der Gegenstände 1 bis 18 umfasst, sowie
   (γ2) therapeutische Behandlung der diagnostizierten Krankheit.

### BEISPIELE

In den folgenden Beispielen wurden miRNAs in einen zellulären Hintergrund eingespikt.

### Beispiel 1

10⁶ Jurkat-Zellen wurden mit 1 µg miR177 antisense-miRNA gemischt und mit Hilfe von 550 µl eines Lysepuffers beinhaltend 0,5 M NaCl, 1% (v/v) Triton-X-100 lysiert. Nach 10 minütiger Inkubation auf Eis wurden 550 µl saures Phenol hinzugegeben. Nach Vortexen wurde für 5 Minuten bei 20.800 × g zentrifugiert, die wässrige Phase abgenommen und mit 652 µg magnetischen, mit Polyethylenimin beschichteten Partikeln gemischt.

Die Partikel wurden erhalten, indem 4 g Epoxid-funktionalisierter magnetischer Partikel (M-PVA E0x-Partikel der Firma Chemagen AG, Baesweiler, Deutschland) in 50 ml einer 10%igen hochmolekulargewichtigen Polyethyleniminlösung (Sigma-Aldrich, Aldrich Kat. Nr. 40,872-7) in Wasser, pH 10, suspendiert, in einen Rundkolben überführt und unter Rühren für 10 Stunden auf 60°C erhitzt werden. Danach wurde diese Mischung sechsmal mit entsalztem Wasser unter magnetischer Abtrennung gewaschen.

Nach 5 minütigem Schütteln auf einem Plattenschüttler wurde der Überstand verworfen und zweimal mit 500 µl Wasser, das auf den pH 4,7, 5.5, 7.0 bzw. 8.5 eingestellt worden war, gewaschen (Spuren a und b im Gel der Figur 1). Eluiert wurde mit 20 µl eines Puffers beinhaltend 1 Mol/l Tris/Cl, pH 9.5, 400 mMol/l KCl, 100 mMol/l Ammoniumsulfat und 30 Mmol/l MgCl₂. Aliquots der Eluate wurden auf ein 15% Polyacrylamidgel geladen und mit Silbernitrat gefärbt (Spuren c in der Figur 1).

Mit 0,5 Mol/l NaCl als Lysepuffer lassen sich effizient miRNAs aufreinigen, in den Eluaten liegen bei der Elution nur noch miRNAs und tRNAs vor, alle übrigen Nukleinsäurespezies sind durch die Aufreinigungsprozedur abgereichert worden.

### Beispiel 2

10⁶ Jurkat-Zellen wurden mit 1 µg let7a antisense-RNA gemischt, lysiert und an magnetische Partikel gebunden, wie in Beispiel 1 angegeben. Nach zwei Waschschritten mit Wasser wurde mit 20 µl Puffer eluiert, wobei als Elutionspuffer 100 mMol/l NaCl, 250 mMol/l NaCl, 400 mMol/l NaCl, 100 mMol/l KCl, 250 mMol/l KCl und 400 mMol/l KCl verendet wurden. Aliquots der Eluate wurden auf ein 15% Polyacrylamidgel geladen und mit Silbernitrat gefärbt (Figur 2).

Bei diesem Versuch wird deutlich, dass sich Salze in unterschiedlichen Molaritäten zur Elution eignen. Bei Verwendung von NaCl, KCl und LiCl (Daten nicht gezeigt) als Elutionspuffer lassen sich sowohl tRNAs als auch miRNAs in hoher Menge aufreinigen.

### Beispiel 3

Es wurde wie im Beispiel 2 verfahren, wobei als Elutionspuffer Puffer mit 10 bis 100 mMol/l MgCl₂ eingesetzt wurden. Aliquots der Eluate wurden auf ein 15% Polyacrylamidgel geladen und mit Silbernitrat gefärbt (Figur 3).

Dieser Versuch zeigt, dass sich auch mit Hilfe von MgCl₂ als Elutionspuffer miRNAs aufreinigen lassen. Werden niedrige Molaritäten von MgCl₂ als Elutionspuffer verwendet, werden die tRNAs sowie längere Nukleinsäuren relativ abgereichert, während die miRNAs mit sehr guter Ausbeute wieder gefunden werden können.

### Beispiel 4

Es wurde wie im Beispiel 2 verfahren, wobei als Elutionspuffer Puffer mit 10 bis 85 mMol/l CaCl₂ eingesetzt wurden. Aliquots der Eluate wurden auf ein 15% Polyacrylamidgel geladen und mit Silbernitrat gefärbt (Figur 4).

Bis zu einer Molarität von CaCl₂ von etwa 50 mMol/l lassen sich miRNAs mit sehr guter Wiederfindungsrate eluieren, während nur Spuren von tRNAs in den Eluaten vorhanden sind. Wird die Molarität weiter erhöht, lassen sich auch tRNAs zusätzlich mit guter Wiederfindungsrate eluieren.

### Beispiel 5

Es wurde wie im Beispiel 2 verfahren, wobei als Elutionspuffer Puffer mit 25 bis 400 mMol/l Ammoniumsulfat bzw. 25 bis 400 mMol/l Ammoniumchlorid eingesetzt wurden. Aliquots der Eluate wurden auf ein 15% Polyacrylamidgel geladen und mit Silbernitrat gefärbt (Figur 5).

Zusammen mit Kalziumchlorid zeigen vor allem die Ammoniumsalze bei der Elution die besten Eigenschaften, um eine hohe miRNA Ausbeute mit gleichzeitig möglichst geringer tRNA Ausbeute zu erzielen. Bis zu einer Ammoniumsalz-Konzentration im Eluat bis etwa 170 bis 200 mMol/l bleibt die Ausbeute an tRNA relativ niedrig, während die Ausbeute an miRNA bei diesen Molaritäten sehr gut ist. Ab einer Konzentration von etwa 400 mMol/l ist auch sehr viel tRNA in den Eluaten zu finden.

## Patentansprüche

1. Ein Verfahren zur Anreicherung von Nukleinsäuren mit einer Länge von weniger als 300 Nukleotiden, umfassend die Verfahrensschritte
i) Bereitstellen einer fluiden, vorzugsweise wässrigen, Phase P₁ beinhaltend
(α1) mindestens eine Nukleinsäure mit einer Länge von weniger als 300 Nukleotiden, sowie
(α2) mindestens eine von dieser Nukleinsäure (α1) verschiedene Komponente,
ii) in Kontakt bringen der Phase P₁ mit einer Anionenaustausch-Matrix zum Anbinden der Nukleinsäure (α1) an die Anionenaustausch-Matrix,
iii) gegebenenfalls Waschen der Anionenaustausch-Matrix mit einem Waschpuffer, wobei die Nukleinsäure (α1) an der Anionenaustausch-Matrix gebunden bleibt, sowie
iv) Abtrennen, vorzugsweise Eluieren, der an der Anionenaustausch-Matrix gebundenen Nukleinsäure (α1) von der Anionenaustausch-Matrix unter Erhalt einer fluiden, vorzugsweise wässrigen, Phase P₂ beinhaltend die Nukleinsäure (α1).

2. Verfahren nach Anspruch 1, wobei es sich bei der Nukleinsäure (α1) um eine RNA handelt,
wobei optional
(i) die RNA ausgewählt ist aus der Gruppe enthaltend miRNA, pre-miRNA, siRNA, snRNA, snoRNA, tRNA, 5S-rRNA, 5,8S-rRNA oder Mischungen aus mindestens zwei davon, oder
(ii) es sich bei der RNA um miRNA, pre-miRNA, tRNA oder Mischungen aus miRNA und tRNA handelt.

3. Verfahren nach einem der vorhergehenden Ansprüche, wobei die Nukleinsäuren aus einer Probe angereichter werden, die ausgewählt ist aus der Gruppe bestehend aus einem zellfreien Probenmaterial, Plasma, Serum, eine Körperflüssigkeit, ein Abstrich und einem Zelllysat,
wobei optional die Körperflüssigkeit Blut, Urin, Sperma, Speichel, Cerebrospinalflüssigkeit oder Sputum ist.

4. Verfahren nach einem der vorhergehenden Ansprüche, wobei es sich bei der im Verfahrensschritt i) bereitgestellten fluiden Phase P₁ um ein Material handelt, das ausgewählt ist aus der Gruppe bestehend aus einem zellfreien Probenmaterial, Plasma, Serum, eine Körperflüssigkeit, und einem Zelllysat.

5. Verfahren nach einem der vorhergehenden Ansprüche, wobei die Anionenaustausch-Matrix funktionelle Gruppen ausgewählt aus der Gruppe enthaltend Amino-Gruppen, Phosphin-Gruppen, Hydrazin-Gruppen und Imin-Gruppen aufweist, und/oder
wobei die Anionenaustausch-Matrix in Form einer Beschichtung auf einem Partikel, einem Filter, einer Membran, einem Monolithen, einer Mikrotiterplatte oder anderen Reagiergefäßen vorliegt,
wobei optional der Partikel ein magnetischer, vorzugsweise superparamagnetischer, ferrimagnetischer oder ferromagnetischer Partikel ist.

6. Verfahren nach einem der Ansprüche 1-5, wobei die Anionenaustausch-Matrix in Form einer Beschichtung auf magnetischen Partikeln vorliegt und wobei weiter das Verfahren eines oder mehrere der folgenden Merkmale umfasst:
a) zur Anbindung der Nukleinsäuren wird die mit den Partikeln in Kontakt gebrachte Phase P₁ kontinuierlich bewegt;
b) die magnetischen Partikel werden als magnetische Aggregate von der Phase P₁ abgetrennt,
c) die an der Anionenaustausch-Matrix gebundene Nukleinsäure (α1) wird durch Ersatz der Phase P₁ oder des Waschpuffers mit einem Elutionspuffer von der Anionenaustausch-Matrix eluiert,
d) das Verfahren ist ohne Wechsel des Reaktionsgefäßes durchführbar.

7. Verfahren nach einem der vorhergehenden Ansprüche, wobei das Trennen im Verfahrens schritt iv) durch einen Elutionspuffer erfolgt,
wobei optional als Elutionspuffer eine wässrige Salzlösung eingesetzt wird.

8. Verfahren nach Anspruch 7, wobei der Elutionspuffer eine wässrige Lösung ist beinhaltend Alkalihalogenide, Erdalkalihalogenide, Ammoniumsalze oder Mischungen aus mindestens zwei dieser Salze und
wobei der Elutionspuffer auch Puffersysteme beinhalten kann, und/oder
wobei der Elutionspuffer wasserlösliche Kalziumsalze, vorzugsweise CaCl₂, wasserlösliche Magnesiumsalze, vorzugsweise MgCl₂, wasserlösliche Ammoniumsalze, vorzugsweise Ammoniumsulfat oder Ammoniumchlorid, oder Mischungen aus mindestens zwei dieser Salze beinhaltet.

9. Verfahren nach Anspruch 7 oder 8, **dadurch gekennzeichnet,**
a) **dass** der Elutionspuffer CaCl₂ beinhaltet und eines der folgenden Merkmale aufweist:
aa) das CaCl₂ liegt in einer Konzentration in einem Bereich von 1 bis 1.000 mMol/l vor;
ab) das CaCl₂ liegt in einer Konzentration in einem Bereich von 5 bis 500 mMol/l vor;
ac) das CaCl₂ liegt in einer Konzentration in einem Bereich von 10 bis 100 mMol/l vor; oder
b) **dass** der Elutionspuffer MgCl₂ beinhaltet und eines der folgenden Merkmale aufweist:
ba) das MgCl₂ liegt in einer Konzentration in einem Bereich von 1 bis 1.000 mMol/l vor;
bb) das MgCl₂ liegt in einer Konzentration in einem Bereich von 5 bis 500 mMol/l vor;
bc) das MgCl₂ liegt in einer Konzentration in einem Bereich von 10 bis 100 mMol/l vor; oder
c) **dass** der Elutionspuffer Ammoniumsulfat und/oder Ammoniumchlorid beinhaltet und eines der folgenden Merkmale aufweist:
ca) diese Salze liegen in einer Gesamtkonzentration in einem Bereich von 1 bis 1.000 mMol/l vor;
cb) diese Salze liegen in einer Gesamtkonzentration in einem Bereich von 5 bis 500 mMol/l vor;
cc) diese Salze liegen in einer Gesamtkonzentration in einem Bereich von 20 bis 300 mMol/l vor.

10. Verfahren nach einem oder mehreren der Ansprüche 7 bis 9, **dadurch gekennzeichnet dass** der pH-Wert des Elutionspuffers in einem Bereich von 5 bis 12 liegt.

11. Verfahren nach Anspruch 7, wobei ein Elutionspuffer eingesetzt wird, der ausschließlich Kalziumsalze, vorzugsweise CaCl₂, und/oder Ammoniumsalze, vorzugsweise Ammoniumsulfat und/oder Ammoniumchlorid beinhaltet, wobei optional ein Elutionspuffer eingesetzt wird bestehend aus Wasser und CaCl₂ in einer Konzentration von bis zu 60 mMol/l oder bestehend aus Wasser und Ammoniumsulfat oder Ammoniumchlorid in einer Konzentration von bis zu 170 bis 200 mMol/l.

12. Verfahren nach Anspruch 7, **dadurch gekennzeichnet dass** der Elutionspuffer ausgewählt ist aus:
a) Elutionspuffer EP₁, enthaltend gelöst in Wasser
i) 1 bis 10.000 mMol/l, 10 bis 5.000 mMol/l oder 50 bis 1.000 mMol/l TRIS,
ii) 1 bis 1.000 mMol/l, 5 bis 800 mMol/l oder 10 bis 500 mMol/l eines Alkalisalzes, vorzugsweise NaCl oder KCl,
iii) 1 bis 400 mMol/l, 10 bis 300 mMol/l oder 50 bis 200 mMol/l eines Ammoniumsalzes, vorzugsweise Ammoniumsulfat oder Ammoniumchlorid und
iv) 0,1 bis 200 mMol/l, 0,5 bis 100 mMol/l oder 1 bis 50 mMol/l eines Magnesiumsalzes, vorzugsweise Magnesiumchlorid,
wobei der Elutionspuffer EP₁ vorzugsweise einen pH-Wert in einem Bereich von 7 bis 11, besonders bevorzugt 8 bis 10 aufweist;
b) Elutionspuffer EP₂, enthaltend gelöst in Wasser 1 bis 1.000 mMol/l, 5 bis 500 mMol/l oder 10 bis 100 mMol/l eines Magnesiumsalzes, vorzugsweise Magnesiumchlorid, wobei der Elutionspuffer EP₂ vorzugsweise einen pH- Wert in einem Bereich von 6 bis 10, besonders bevorzugt 7 bis 9 aufweist;
c) Elutionspuffer EP₃, enthaltend gelöst in Wasser 1 bis 1.000 mMol/l, 5 bis 500 mMol/l oder 10 bis 100 mMol/l eines Kalziumsalzes, vorzugsweise Kalziumchlorid wobei der Elutionspuffer EP₃ vorzugsweise einen pH-Wert in einem Bereich von 6 bis 10, besonders bevorzugt 7 bis 9 aufweist;
d) Elutionspuffer EP₄, enthaltend gelöst in Wasser 1 bis 1.000 mMol/l, 5 bis 500 mMol/l oder 20 bis 300 mMol/l eines Ammoniumsalzes, vorzugsweise Ammoniumchlorid oder Ammoniumsulfat, wobei der Elutionspuffer EP₄ vorzugsweise einen pH- Wert in einem Bereich von 6 bis 10, besonders bevorzugt 7 bis 9 aufweist;
e) Elutionspuffer EP₅ enthaltend gelöst in Wasser 1 bis 2.000 mMol/l, 10 bis 1.000 mMol/l oder 100 bis 500 mMol/l eines Alkalisalzes, vorzugsweise Kaliumchlorid, Natriumchlorid oder Lithiumchlorid, wobei der Elutionspuffer EP₅ vorzugsweise einen pH- Wert in einem Bereich von 6 bis 10, besonders bevorzugt 7 bis 9 aufweist.

13. Verfahren nach einem der vorhergehenden Ansprüche, wobei die im Verfahrensschritt i) bereitgestellte, wässrige Phase P₁ ein Zelllysat ist, wobei optional das Zelllysat erhältlich ist durch ein Verfahren umfassend die Verfahrensschritte:
I) Bereitstellen von Zellen,
II) Lyse der Zellen unter Erhalt eines Zelllysates, sowie
III) gegebenenfalls mindestens teilweises Abtrennen der mindestens einen, von der Nukleinsäure (α1) verschiedenen Komponente (α2) aus dem Zelllysat.

14. Verfahren nach einem der Ansprüche 2 bis 13, wobei die relative Menge an RNA mit einer Länge von weniger als 300 Nukleotiden in der Phase P₂, bezogen auf die Gesamtmenge an RNA in der Phase P₂, um einen Faktor von mindestens 2 größer ist als die relative Menge an RNA mit einer Länge von weniger als 300 Nukleotiden in der Phase P₁, bezogen auf die Gesamtmenge an RNA in der Phase P₁, oder
wobei die relative Menge an miRNA in der wässrigen Phase P₂, bezogen auf die Gesamtmenge an miRNA und tRNA in der wässrigen Phase P₂, um einen Faktor von mindestens 2 größer ist als die relative Menge an miRNA in der wässrigen Phase P₁, bezogen auf die Gesamtmenge an miRNA und tRNA in der wässrigen Phase P₁.

15. Verfahren nach einem oder mehreren der Ansprüche 1 bis 14, **dadurch gekennzeichnet, dass** das Anbinden der Nukleinsäure (α1) an die Anionenaustausch-Matrix im Verfahrensschritt ii) in Gegenwart eines Alkalisalzes, vorzugsweise Natriumchlorid, erfolgt, wobei die Konzentration des Alkalisalzes beim Anbinden in einem Bereich von 0,01 bis 10 Mol/l, vorzugsweise 0,05 bis 5 Mol/l, besonders bevorzugt 0,25 bis 0,75 Mol/l liegt, und/oder
**dadurch gekennzeichnet, dass** das Anbinden der Nukleinsäure (α1) an die Anionenaustausch-Matrix im Verfahrensschritt ii) bei einem pH-Wert in einem Bereich von 2 bis 7 oder von 4 bis 6 erfolgt.

16. Ein Kit zur Anreicherung von Nukleinsäuren mit einer Länge von weniger als 300 Nukleotiden, umfassend:
(β1) einen Lysepuffer oder ein Lysepufferkonzentrat,
(β2) eine Anionenaustausch-Matrix,
(β3) einen Elutionspuffer, wobei optional der Elutionspuffer ein Elutionspuffer gemäß Anspruch 12 ist,
(β4) gegebenenfalls einen Suspensionspuffer,
(β5) gegebenenfalls einen Neutralisationspuffer,
(β6) gegebenenfalls einen Waschpuffer sowie
(β7) gegebenenfalls eine Extraktionssubstanz.
wobei die Anionenaustausch-Matrix (β2) optional:
a) funktionelle Gruppen ausgewählt aus der Gruppe beinhaltend Amino-Gruppen, Phosphin-Gruppen, Hydrazin-Gruppen und Imin-Gruppen aufweist; und/oder
b) in Form einer Beschichtung auf einem Partikeln, einem Filter, einer Membran, einem Monolithen oder einer Mikrotiterplatte vorliegt; und/oder
c) in Form einer Beschichtung auf einem magnetischen, vorzugsweise einem superparamagnetischen, ferrimagnetischen oder ferromagnetischen Partikel vorliegt.

17. Verwendung des Kits nach Anspruch 16 zur Anreicherung von Nukleinsäuren mit einer Länge von weniger als 300 Nukleotiden, wobei optional das Kit in einem Verfahren nach einem der Ansprüche 1 bis 15 verwendet wird.

18. Verwendung einer Anionenaustausch-Matrix zur Anreicherung von Nukleinsäuren mit einer Länge von weniger als 300 Nukleotiden.
